# EUROPEAN PATENT APPLICATION

(11) **EP 1 607 098 A1**
(43) Date of publication of application: **21.12.2005**
(21) Application number: 04291348.3
(22) Date of filing: 28.05.2004
(51) Int. Cl.: A61K 35/78, A61K 7/40, A61K 7/48, A61P 17/16

(54) **Cosmetic use of an active ingredient that increases Gadd45 gene expression**

(71) Applicant: Cognis France, S.A.S., 31360 Boussens (FR)
(72) Inventor: Bardey, Vincent, 54000 Nancy (FR); Danoux, Louis, 54420 Saulxures les Nancy (FR); Pauly, Gilles, 54000 Nancy (FR); Jeanmaire, Christine, 54000 Nancy (FR); Henry, Florence, 54600 Villers-Les-Nancy (FR)
(74) Representative: Gittinger, Andreas

(57) **Abstract**

The present invention relates to the use of an active ingredient that increases gadd45 gene expression in mammalian skin (preferably human skin) for the manufacture of a cosmetic composition. Furthermore the present invention relates to the use of an active ingredient that increases gadd45 gene expression in mammalian skin (preferably human skin) for the cosmetic treatment of human skin.

## Description

The present invention relates to the use of an active ingredient that increases gadd45 gene expression in mammalian skin (preferably human skin) for the manufacture of a cosmetic composition. Furthermore the present invention relates to the use of an active ingredient that increases gadd45 gene expression in mammalian skin (preferably human skin) for the cosmetic treatment of human skin.

The growth arrest and DNA damage-inducible (gadd45) gene family is composed of three genes (gadd45α/DDIT-1, gadd45β/MyD118 and gadd45γ/DDIT2/GRP17/GADD-related protein17kD/CR6/OIG37), based on a high conservation of their amino acid sequences (NAKAYAMA K., HARA T., HIBI M., HIRANO T. and MIYAJIMA A.: A novel oncostatin M-inducible gene OIG37 forms a gene family with MyD118 and GADD45 and negatively regulates cell growth, J. Biol. Chem., 1999, volume 274, pages 24766 to 24772).

These genes are identified as early and highly induced genes in mammalian cells after stress. These three genes are differently regulated. Indeed, the kinetics and the amplitude of induction of these three genes are different according to the applied stress, such as UV-C radiation or chemical agents inducing DNA damages (WANG X., GOROSPE M. And HOLBROOK N.J.: gadd45 is not required for activation of c-Jun N-terminal kinase or p38 during acute stress, J. Biol. Chem., 1999, volume 274, pages 29599 to 29602).

In addition, only the gadd45α gene is induced by IR radiation, whereas the three genes are induced by different molecular mechanisms after UV radiation (HOLLANDER M.C., ALAMO I., JACKMAN J., WANG M.G. and MCBRIDE O.W.: Analysis of the mammalian gadd45 gene and its response to DNA damage. J. Biol. Chem., 1993, volume 268, pages 24385 to 24393 ; GRAUNKE D.M., FORNACE A.J. and PIEPER R.O.: Presetting of chromatin structure and transcription factor binding poise the human GADD45 gene for rapid transcriptional up-regulation. Nucl. Acids Res., 1999, volume 27, pages 3881 to 3890 ; JIN S., FAN F., ZHAO H., TONG T., BLANCK P., ALOMO I., RAJASEKARAN B. and ZHAN Q.: Transcription factors Oct-1 and NF-YA regulate the p53-independent induction of the GADD45 following DNA damage. Oncogene, 2001, volume 20, pages 2683 to 2690).

These three genes have, at least in part, redundant functions, and are implicated in the cellular response against stress. The gadd45 genes affect 3 different pathways required to an efficient DNA repair:
- the arrest of cell growth, to allow the repair of the DNA before cell division
- the accessibility of the modified DNA to repair machineries
- the efficiency of the DNA repair systems

The Gadd45 proteins are important cell-cycle check-point proteins that arrest cells at the G2-M phase cycle by inhibiting the activity of the G2-specific complex cyclinB-cdc2 (MAEDA T., HANNA A.N., SIM A.B., CHUA P.P., CHONG M.T. and TRON V.A.: GADD45 regulates G2/M arrest, DNA repair, and cell death in keratinocytes following ultraviolet exposure. J. Invest. Dermatol., 2002, volume 119, pages 22 to 26). This allows to increase the time needed to repair DNA damages.

In addition, the Gadd45 proteins interact through their acidic function with UV-altered nucleosomes. This interaction modifies the structure of the DNA chromatin to increase the accessibility of altered-DNA nucleotides to trans proteins including the DNA repair machinery (CARRIER F., GEORGEL P.T., POURQUIER P., BLAKE M., KONTNY H.U., ANTINORE M.J., GARIBOLDI M., MYERS T.G., WEINSTEIN J.N., POMMIER Y. and FORNACE A.J. Jr.: Gadd45, a p53-responsive stress protein, modifies DNA accessibility on damaged chromatin., Mol. Cell. Biol., 1999, volume 19, pages 1673 to 1685).

Moreover, the Gadd45-encoded proteins interact with a key protein, the proliferating cell nuclear antigen (PCNA). This interaction is required for DNA repair activation in vitro (SMITH M.L., CHEN I.T., ZHAN Q., BAE I., CHEN C.Y., GILMER T.M., KASTAN M.B., O'CONNOR P.M. and FORNACE A.J. Jr.: Interaction of the p53-regulated protein Gadd45 with proliferating cell nuclear antigen, Science, 1994, volume 266, pages 1376 to 1380).

The molecular mechanism of the gadd45 activation has not been identified yet. However, PCNA is required for the efficiency of DNA polymerases, implicated in both DNA replication and repair and belongs to a large complex containing many proteins of the different repair pathways (PAUNESKU T., MITTAL S., PROTIC M., ORYHON J., KOROLEV S.V., JOACHIMIAK A. and WOLOSCHAK G.E.: Proliferating cell nuclear antigen (PCNA): ringmaster of the genome., Int. J. Radiat. Biol., 2001, volume 77, pages 1007 to 1021). The interaction between PCNA and Gadd45 protein may increase the activity of PCNA.

As the gadd45 genes are implicated in the activation of the DNA repair efficiency, they strongly modify the cellular survey after genotoxic stress (SMITH M.L., FORD J.M., HOLLANDER M.C., BORTNICK R.A., AMUNDSON S.A., SEO Y.R., DENG C.X., HANAWALT P.C. and FORNACE A.J. Jr.: p53-mediated DNA repair responses to UV radiation: studies of mouse cells lacking p53, p21, and/or gadd45 genes, Mol. Cell. Biol., 2000, volume 20, pages 3705 to 3714). The gadd45 proteins may act as signals to boost the pathways for an efficient DNA repair.

Some cell treatments (e. g. oligonucleotides that mime DNA damages) do not induce real DNA alteration, but increase expression of some genes, including gadd45α (ELLER M.S., MAEDA T., MAGNONI C., ATWAL D. and GILCHREST B.A.: Enhancement of DNA repair in human skin cells by thymidine dinucleotides: evidence for a p53-mediated mammalian SOS response, Proc. Natl. Acad. Sci. USA, 1997, volume 94, pages 12627 to 12632). This oligonucleotide treatment boosts the DNA repair activities, and allows to increase the resistance of cells to further-applied stress, illustrating the role of gadd45 genes in a cell DNA repair adaptive response.

In addition an age-associated decrease of DNA repair capacities after UV radiation has been observed. This is in part due to a decrease of the DNA repair and to a reduction of the gadd45 gene induction (EDWARDS M.G., SARKAR D., KLOPP R., MORROW J.D., WEINDRUCH R; and PROLLA T.A.: Age-related impairment of the transcriptional responses to oxidative stress in the mouse heart, Physiol. Genomics, 2003, volume 13, pages 119 to 127).

Oligonucleotide treatment of old cells, boosting gadd45α gene expression, allows to correct the age-associated decline of DNA repair (GOUKASSIAN D., GAD F., YAAR M., ELLER M.S., NEHAL U.S. and GILCHREST B.A:. Mechanisms and implications of the age-associated decrease in DNA repair capacity, FASEB J., 2000, volume 14, pages 1325 to 1334).

These observations confirm the high implication of gadd45 genes in the efficiency of the DNA repair after a stress, the major role of the loss of gadd45 induction in the age-associated decline of DNA repair and the importance of the gadd45 gene induction in cell pre-conditioning.

The present invention has revealed that extracts of the plant Cassia alata can increase the gadd45 gene expression. WO 02/17938 discloses the cosmetic use of extracts of the plant Cassia alata.

The problem underlying the present invention is to find substances that can be used for the cosmetic treatment of human skin.

This problem is solved by the use of an active ingredient that increases gadd45 gene expression in mammalian skin (preferably human skin) for the manufacture of a cosmetic composition. This use is a subject of the present invention.

It is furthermore solved by the use of an active ingredient that increases gadd45 gene expression in mammalian skin (preferably human skin) or for the cosmetic treatment of human skin and/or hair. This use is a subject of the present invention.

One embodiment of the present invention is the use according to the present invention, wherein gadd45 gene expression is the expression of at least one gene selected from the group consisting of gadd45α, gadd45β and gadd45γ.

Another embodiment of the present invention is the use according to the present invention, wherein the active ingredient is an aqueous extract of the leaves of the plant Cassia alata.

Another embodiment of the present invention is the use according to the present invention, wherein the aqueous extract of the leaves of the plant Cassia alata is obtainable by extracting the leaves of the plant Cassia alata with water and removing the water in order to obtain the extract.

Another embodiment of the present invention is the use according to the present invention, wherein the active ingredient is used in a composition comprising the active ingredient, glycerol, chlorphenesin, methylparaben and water.

Another embodiment of the present invention is the use according to the present invention, wherein the active ingredient is used in a composition comprising the active ingredient and at least one cosmetic adjuvant.

Another embodiment of the present invention is the use according to the present invention, wherein the cosmetic adjuvant is selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

Another embodiment of the present invention is the use according to the present invention, wherein the cosmetic treatment of human skin comprises the protection of the skin against ageing or against photoageing or against UV radiation or against pollutants or against IR radiation or against stress in general or against inflammation or against irritation or against sensitization or against a weakening of the immune system.

Another embodiment of the present invention is the use according to the present invention, wherein the active ingredient that increases gadd45 gene expression is selected from the group consisting of derivatives of kaempferol (such as kaempferol 3-O-sophoroside), lignans, tannins (such as catechuic tannins), retinoids, derivatives of retinoid, vitamin D and derivatives of vitamin D.

The increase of gadd45 gene expression stimulates the cellular DNA repair capacities.

There is state of the art that discloses aspects of DNA repair. However, the gadd45 pathway has not been disclosed yet.

US-A 6 403 565A discloses a method of improving DNA repair by administering to the skin a composition including individual deoxyribonucleoside or deoxyribonucleotide.

WO 01/74342discloses a method of reducing photoaging of skin by treating the skin compositions containing oligonucleotides and dinucleotides.

The gadd45 pathway represents a key step in DNA repair and allows the damaged DNA to be repaired properly.

The precise evaluation of the expression of the three existing *gadd45* genes needs to quantify precisely the levels of mRNAs or proteins encoded by each of the three *gadd45α,* β and γ genes, while previous studies have measured the activity of dinucleotides only on the *gadd45α* gene expression (ELLER M.S., MAEDA T., MAGNONI C., ATWAL D. and GILCHREST B.A.: Enhancement of DNA repair in human skin cells by thymidine dinucleotides: evidence for a p53-mediated mammalian SOS response, Proc. Natl. Acad. Sci. USA, 1997, volume 94, pages 12627 to 12632).

The present invention concerns the cosmetic use of an active ingredient that stimulates the cellular DNA repair capacities by increasing the gadd45 gene expression.

An aqueous extract of the leaves of the plant Senna alata (L.) Roxb (synonym: Cassia alata L.) is one embodiment of the active ingredient according to the present invention. The active ingredient according to the present invention can induce the gadd45=pathway with or without UV-B irradiation, in order to increase the efficiency of the DNA repair system of the cells of skin.

The present invention also concerns the cosmetic use of an active ingredient that stimulates cellular DNA repair by increasing the gadd45α/DDIT-1 and/or gadd45β/MyD118 and/or gadd45γ/DDIT2/GRP17/GADD-related protein17kD/CR6/OIG37 as cell defences booster.

The present invention also concerns the cosmetic use of an active ingredient that stimulates the cellular DNA repair by increasing the gadd45α/DDIT-1 and/or gadd45β/MyD118 and/or gadd45γ/DDIT2/GRP17/GADD-related protein17kD/CR6/OIG37 in order to protect the skin immune system.

The present invention also concerns the cosmetic use of an active ingredient that stimulates the cellular DNA repair by increasing the gadd45α/DDIT-1 and/or gadd45β/MyD118 and/or gadd45γ/ DDIT2/GRP17/GADD-related protein17kD/CR6/OIG37 in order to fight against stress such as UV irradiation, IR irradiation, pollution and oxidative stress such as inflammation, irritation, sensitization etc.

The present invention also concerns the cosmetic use of an active ingredient that stimulates the cellular DNA repair by increasing the gadd45α/DDIT-1 and/or gadd45β/MyD118 and/or gadd45γ/ DDIT2/GRP17/GADD-related protein 17kD/CR6/OIG37 in order to fight against aging or photoaging.

The present invention also concerns the cosmetic use of an aqueous extract of leaves of Senna alata that stimulates the cellular DNA repair by increasing the gadd45α/DDIT-1 and/or gadd45β/MyD118 and/or gadd45γ/ DDIT2/GRP17/GADD-related protein17kD/CR6/OIG37 as cell defences booster.

The present invention also concerns the cosmetic use of an aqueous extract of leaves of Senna alata that stimulates the cellular DNA repair by increasing the gadd45α/DDIT-1 and/or gadd45β/MyD118 and/or gadd45γ/DDIT2/GRP17/GADD-related protein17kD/CR6/OIG37 in order to protect the skin immune system.

The present invention also concerns the cosmetic use of an aqueous extract of leaves of Senna alata that stimulates the cellular DNA repair by increasing the gadd45α/DDIT-1 and/or gadd45β/MyD118 and/or gadd45γ/ DDIT2/GRP17/GADD-related protein17kD/CR6/OIG37 in order to fight against aging or photoaging.

The present invention also concerns the cosmetic use of an aqueous extract of leaves of Senna alata that stimulates the cellular DNA repair by increasing the gadd45α/DDIT-1 and/or gadd45β/MyD118 and/or gadd45γ/ DDIT2/GRP17/GADD-related protein17kD/CR6/OIG37 in order to fight against stress such as UV irradiation, IR irradiation, pollution and oxidative stress such as inflammation, irritation, sensitisation etc.

The cosmetic adjuvant of the present invention can be selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

In one embodiment of the present invention the cosmetic adjuvant is selected from the group consisting of surfactants, emulsifiers, fats, waxes, stabilizers, deodorants, antiperspirants, antidandruff agents and perfume oils.

The total content of the cosmetic adjuvant may be 1 to 50% by weight, preferably 5 to 40% by weight, based on the cosmetic preparations (cosmetic compositions). The preparations can be prepared by customary cold or hot processes; preference is given to using the phase-inversion temperature method.

For the purposes of the invention, cosmetic preparations can mean care agents. Care agents are understood as meaning care agents for skin and hair. These care agents include, inter alia, cleansing and restorative action for skin and hair.

Application can be topical or oral in the form of tablets, dragees, capsules, juices, solutions and granules.

The compositions and cosmetic preparations according to the invention can be used for the preparation of cosmetic and/or dermopharmaceutical preparations, e. g. hair shampoos, hair lotions, foam baths, shower baths, creams, gels, lotions, alcoholic and aqueous/alcoholic solutions, emulsions, wax/fat compositions, stick preparations, powders or ointments. Furthermore, the preparations for oral application according to the invention can also be incorporated into tablets, dragees, capsules, juices, solutions and granules.

Surfactants (or Surface-active substances) that may be present are anionic, non-ionic, cationic and/or amphoteric or amphoteric surfactants, the content of which in the compositions is usually about 1 to 70% by weight, preferably 5 to 50% by weight and in particular 10 to 30% by weight. Typical examples of anionic surfactants are soaps, alkylbenzenesulfonates, alkanesulfonates, olefin sulfonates, alkyl ether sulfonates, glycerol ether sulfonates, α-methyl ester sulfonates, sulfo fatty acids, alkyl sulphates, fatty alcohol ether sulphates, glycerol ether sulphates, fatty acid ether sulphates, hydroxy mixed ether sulphates, monoglyceride (ether) sulphates, fatty acid amide (ether) sulphates, mono- and dialkyl sulfosuccinates, mono- and dialkyl sulfo-succinamates, sulfotriglycerides, amide soaps, ether carboxylic acids and salts thereof, fatty acid isethionates, fatty acid sarcosinates, fatty acid taurides, N-acylamino acids, e. g. acyl lactylates, acyl tartrates, acyl glutamates and acyl aspartates, alkyl oligoglucoside sulphates, protein fatty acid condensates (in particular wheat-based vegetable products) and alkyl (ether) phosphates. If the anionic surfactants contain polyglycol ether chains, these may have a conventional homologous distribution, but preferably have a narrowed homologous distribution. Typical examples of non-ionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers or mixed formals, optionally partially oxidized alk(en)yl oligoglycosides or glucoronic acid derivatives, fatty acid N-alkylglucamides, protein hydrolysates (in particular wheat-based vegetable products), polyol fatty acid esters, sugar esters, sorbitan esters, polysorbates and amine oxides. If the non-ionic surfactants contain polyglycol ether chains, these may have a conventional homologous distribution, but preferably have a narrowed homologous distribution. Typical examples of cationic surfactants are quaternary ammonium compounds, e. g. dimethyldistearylammonium chloride, and ester quats, in particular quaternized fatty acid trialkanolamine ester salts. Typical examples of amphoteric or zwitterionic surfactants are alkylbetaines, alkylamidobetaines, aminopropionates, aminoglycinates, imidazolinium-betaines and sulfobetaines. Said surfactants are known compounds. With regard to structure and preparation of these substances, reference may be made to relevant review works.

Typical examples of particularly suitable mild, i.e. particularly skin-compatible surfactants are fatty alcohol polyglycol ether sulphates, monoglyceride sulphates, mono-and/or dialkyl sulfosuccinates, fatty acid isethionates, fatty acid sarcosinates, fatty acid taurides, fatty acid glutamates, α-olefinsulfonates, ether carboxylic acids, alkyl oligoglucosides, fatty acid glucamides, alkylamidobetaines, amphoacetals and/or protein fatty acid condensates, the latter preferably based on wheat proteins.

Suitable oily bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, for example myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkylhydroxy-carboxylic acids with linear or branched C₆-C₂₂-fatty alcohols, in particular dioctyl malates, esters of linear and/or branched fatty acids with polyhydric alcohols (for example propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, for example dicaprylyl carbonates (Cetiol® CC), Guerbet carbonates based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or unsymmetrical dialkyl ethers having 6 to 22 carbon atoms per alkyl group, for example dicaprylyl ether (Cetiol® OE), ring-opening products of epoxidized fatty acid esters with polyols, silicone oils (cyclomethicones, silicon methicone types, inter alia) and/or aliphatic or naphthenic hydrocarbons, for example squalane, squalene or dialkylcyclohexanes.

Suitable emulsifiers are, for example, nonionogenic surfactants from at least one of the following groups:
- addition products of from 2 to 30 mol of ethylene oxide and/or 0 to 5 mol of propylene oxide onto linear fatty alcohols having 8 to 22 carbon atoms, onto fatty acids having 12 to 22 carbon atoms, onto alkylphenols having 8 to 15 carbon atoms in the alkyl group, and onto alkylamines having 8 to 22 carbon atoms in the alkyl radical;
- alkyl and/or alkenyl oligoglycosides having 8 to 22 carbon atoms in the alk(en)yl radical and the ethoxylated analogs thereof;
- addition products of from 1 to 15 mol of ethylene oxide onto castor oil and/or hydrogenated castor oil;
- addition products of from 15 to 60 mol of ethylene oxide onto castor oil and/or hydrogenated castor oil;
- partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide;
- partial esters of polyglycerol (average degree of self-condensation 2 to 8), polyethylene glycol (molecular weight 400 to 5 000), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide;
- mixed esters of pentaerythritol, fatty acids, citric acid and fatty alcohols and/or mixed esters of fatty acids having 6 to 22 carbon atoms, methylglucose and polyols, preferably glycerol or polyglycerol,
- mono-, di- and trialkyl phosphates, and mono-, di- and/or tri-PEG alkyl phosphates and salts thereof;
- wool wax alcohols;
- polysiloxane-polyalkyl-polyether copolymers and corresponding derivatives;
- block copolymers, e.g. polyethylene glycol-30 dipolyhydroxystearates;
- polymer emulsifiers, e.g. Pemulen® grades (TR-1, TR-2) from Goodrich;
- polyalkylene glycols and
- glycerol carbonate.

The addition products of ethylene oxide and/or of propylene oxide onto fatty alcohols, fatty acids, alkylphenols or onto castor oil are known, commercially available products. These are homologous mixtures whose average degree of alkoxylation corresponds to the ratio of the amounts of ethylene oxide and/or propylene oxide and substrate with which the addition reaction is carried out. C_{12/18}-fatty acid mono- and diesters of addition products of ethylene oxide onto glycerol are known as refatting agents for cosmetic preparations.

Alkyl and/or alkenyl oligoglycosides, their preparation and their use are known from the prior art. They can be prepared by reacting glucose or oligosaccharides with primary alcohols having 8 to 18 carbon atoms. With regard to the glycoside radical, both monoglycosides, in which a cyclic sugar radical is glycosidically bonded to the fatty alcohol, and also oligomeric glycosides having a degree of oligomerization of up to, preferably, about 8, are suitable. The degree of oligomerization here is a statistical average value that is based on a homologous distribution customary for such technical-grade products.

Typical examples of suitable partial glycerides are hydroxy stearic acid monoglyceride, hydroxy stearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linoleic acid monoglyceride, linoleic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride, and the technical-grade mixtures thereof which may also comprise small amounts of triglyceride as a minor product of the preparation process. Likewise suitable are addition products of 1 to 30 mol, preferably 5 to 10 mol, of ethylene oxide onto said partial glycerides.

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate, and technical-grade mixtures thereof. Likewise suitable are addition products of 1 to 30 mol, preferably 5 to 10 mol, of ethylene oxide onto said sorbitan esters.

Typical examples of suitable polyglycerol esters are polyglyceryl-2 dipolyhydroxystearate (Dehymuls® PGPH), polyglycerol-3 diisostearate (Lameform® TGI), polyglyceryl-4 isostearate (Isolan® GI 34), polyglyceryl-3 oleate, diisostearoyl polyglyceryl-3 diisostearate (Isolan® PDI), polyglyceryl-3 methylglucose distearate (Tego Care® 450), polyglyceryl-3 beeswax (Cera Bellina®), polyglyceryl-4 caprate (Polyglycerol Caprate T2010/90), polyglyceryl-3 cetyl ether (Chimexane® NL), polyglyceryl-3 distearate (Cremophor® GS 32) and polyglyceryl polyricinoleate (Admul® WOL 1403), polyglyceryl dimerate isostearate, and mixtures thereof. Examples of further suitable polyol esters are the mono-, di- and triesters, optionally reacted with 1 to 30 mol of ethylene oxide, of trimethylolpropane or pentaerythritol with lauric acid, coconut fatty acid, tallow fatty acid, palmitic acid, stearic acid, oleic acid, behenic acid and the like.

Furthermore, zwitterionic surfactants can be used as emulsifiers. The term "zwitterionic surfactants" refers to those surface-active compounds that carry at least one quaternary ammonium group and at least one carboxylate and one sulfonate group in the molecule. Particularly suitable zwitterionic surfactants are the betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines having in each case 8 to 18 carbon atoms in the alkyl or acyl group, and cocoacylaminoethylhydroxyethylcarboxymethyl glycinate. Particular preference is given to the fatty acid amide derivative known under the CTFA name *Cocamidopropyl Betaine.* Likewise suitable emulsifiers are ampholytic surfactants. The term "ampholytic surfactants" means those surface-active compounds that, apart from a C_{8/18}-alkyl or -acyl group in the molecule, contain at least one free amino group and at least one -COOH or -SO₃H group and are capable of forming internal salts. Examples of suitable ampholytic surfactants are N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkyl-aminopropionic acids and alkylaminoacetic acids having in each case about 8 to 18 carbon atoms in the alkyl group. Particularly preferred ampholytic surfactants are N-cocoalkyl aminopropionate, cocoacylaminoethyl aminopropionate and C_{12/18}-acyl-sarcosine. Finally, cationic surfactants are also suitable emulsifiers, those of the ester quat type, preferably methyl-quaternized difatty acid triethanolamine ester salts, being particularly preferred.

Fats and waxes that can be used are described in the following text. Typical examples of fats are glycerides, i.e. solid or liquid vegetable or animal products which consist essentially of mixed glycerol esters of higher fatty acids, suitable waxes are inter alia natural waxes, for example candelilla wax, carnauba wax, japan wax, esparto grass wax, cork wax, guaruma wax, rice germ oil wax, sugarcane wax, ouricury wax, montan wax, beeswax, shellac wax, spermaceti, lanolin (wool wax), uropygial grease, ceresin, ozokerite (earth wax), petrolatum, paraffin waxes, microcrystalline waxes; chemically modified waxes (hard waxes), for example montan ester waxes, sasol waxes, hydrogenated jojoba waxes, and synthetic waxes, for example polyalkylene waxes and polyethylene glycol waxes. In addition to the fats, suitable additives are also fat-like substances, such as lecithins and phospholipids. The term lecithins is understood by the person skilled in the art as meaning those glycerophospholipids which form from fatty acids, glycerol, phosphoric acid and choline by esterification. Lecithins are thus frequently also [lacuna] as phosphatidylcholines (PC). Examples of natural lecithins which may be mentioned are the cephalins, which are also referred to as phosphatidic acids and represent derivatives of 1,2-diacyl-sn-glycerol-3-phosphoric acids. By contrast, phospholipids are usually understood as meaning mono- and, preferably, diesters of phosphoric acid with glycerol (glycerophosphates), which are generally considered to be fats. In addition, sphingosines and sphingolipids are also suitable.

Examples of suitable pearlescent waxes are: alkylene glycol esters, specifically ethylene glycol distearate; fatty acid alkanolamides, specifically coconut fatty acid diethanolamide; partial glycerides, specifically stearic acid monoglyceride; esters of polybasic, optionally hydroxy-substituted carboxylic acids with fatty alcohols having 6 to 22 carbon atoms, specifically long-chain esters of tartaric acid; fatty substances, for example fatty alcohols, fatty ketones, fatty aldehydes, fatty ethers and fatty carbonates, which have a total of at least 24 carbon atoms, specifically laurone and distearyl ether; fatty acids, such as stearic acid, hydroxystearic acid or behenic acid, ring-opening products of olefin epoxides having 12 to 22 carbon atoms with fatty alcohols having 12 to 22 carbon atoms and/or polyols having 2 to 15 carbon atoms and 2 to 10 hydroxyl groups, and mixtures thereof.

Bodying agents and thickeners that can be used are described in the following text. Suitable bodying agents are primarily fatty alcohols or hydroxy fatty alcohols having 12 to 22, and preferably 16 to 18, carbon atoms, and also partial glycerides, fatty acids or hydroxy fatty acids. Preference is given to a combination of these substances with alkyl oligoglucosides and/or fatty acid N-methylglucamides of identical chain length and/or polyglycerol poly-12-hydroxystearates. Suitable thickeners are, for example, Aerosil grades (hydrophilic silicas), polysaccharides, in particular xanthan gum, guar guar, agar agar, alginates and Tyloses, carboxymethylcellulose and hydroxyethylcellulose, and also relatively high molecular weight polyethylene glycol mono- and diesters of fatty acids, polyacrylates (e.g. Carbopols® and Pemulen grades from Goodrich; Synthalens® from Sigma; Keltrol grades from Kelco; Sepigel grades from Seppic; Salcare grades from Allied Colloids), polyacrylamides, polymers, polyvinyl alcohol and polyvinylpyrrolidone, surfactants, for example ethoxylated fatty acid glycerides, esters of fatty acids with polyols for example pentaerythritol or trimethylolpropane, fatty alcohol ethoxylates having a narrowed homolog distribution or alkyl oligoglucosides, and electrolytes such as sodium chloride and ammonium chloride.

Superfatting agents which can be used are for example lanolin and lecithin, and polyethoxylated or acylated lanolin and lecithin derivatives, polyol fatty acid esters, monoglycerides and fatty acid alkanolamides, the latter also serving as foam stabilizers.

Stabilizers which can be used are metal salts of fatty acids, for example magnesium, aluminium and/or zinc stearate or ricinoleate.

Polymers that can be used are described in the following text. Suitable cationic polymers are, for example, cationic cellulose derivatives, for example a quaternized hydroxyethylcellulose obtainable under the name Polymer JR 400® from Amerchol, cationic starch, copolymers of diallylammonium salts and acryl amides, quaternized vinylpyrrolidone-vinylimidazole polymers, for example Luviquat® (BASF), condensation products of polyglycols and amines, quaternized collagen polypeptides, for example lauryldimonium hydroxypropyl hydrolysed collagen (Lamequat®L/Grünau), quaternized wheat polypeptides, polyethyleneimine, cationic silicone polymers, for example amodimethicones, copolymers of adipic acid and dimethylaminohydroxypropyldiethylenetriamine (Cartaretins®/Sandoz), copolymers of acrylic acid with dimethyl diallylammonium chloride (Merquat® 550/Chemviron), polyaminopolyamides and cross linked water-soluble polymers thereof, cationic chitin derivatives, for example quaternized chitosan, optionally in microcrystalline dispersion, condensation products from dihaloalkyls, for example dibromobutane with bisdialkylamines, for example bis-dimethylamino-1,3-propane, cationic guar gum, for example Jaguar® CBS, Jaguar® C-17, Jaguar® C-16 from Celanese, quaternized ammonium salt polymers, for example Mirapol® A-15, Mirapol® AD-1, Mirapol® AZ-1 from Miranol.

Suitable anionic, zwitterionic, amphoteric and nonionic polymers are, for example, vinyl acetate-crotonic acid copolymers, vinylpyrrolidone-vinyl acrylate copolymers, vinyl acetate-butyl maleate-isobornyl acrylate copolymers, methyl vinyl ether-maleic anhydride copolymers and esters thereof, uncrosslinked polyacrylic acids and polyacrylic acids crosslinked with polyols, acrylamidopropyltrimethylammonium chloride-acrylate copolymers, octylacrylamide-methyl methacrylate-tert-butylaminoethyl methacrylate-2-hydroxypropyl methacrylate copolymers, polyvinylpyrrolidone, vinylpyrrolidone-vinyl acetate copolymers, vinylpyrrolidone-dimethylaminoethyl methacrylate-vinylcaprolactam terpolymers, and optionally derivatized cellulose ethers and silicones.

Suitable silicone compounds are, for example, dimethylpolysiloxanes, methylphenylpolysiloxanes, cyclic silicones, and amino-, fatty-acid-, alcohol-, polyether-, epoxy-, fluorine-, glycoside- and/or alkyl-modified silicone compounds, which can either be liquid or in resin form at room temperature. Also suitable are simethicones, which are mixtures of dimethicones having an average chain length of from 200 to 300 dimethylsiloxane units and hydrogenated silicates.

Deodorants and antimicrobial agents that can be used are described in the following text. Cosmetic deodorants counteract, mask or remove body odors. Body odors arise as a result of the effect of skin bacteria on apocrine perspiration, with the formation of degradation products which have an unpleasant odor. Accordingly, deodorants comprise active ingredients which act as antimicrobial agents, enzyme inhibitors, odor absorbers or odor masking agents. Suitable antimicrobial agents are, in principle, all substances effective against gram-positive bacteria, for example 4-hydroxybenzoic acid and its salts and esters, N-(4-chlorophenyl)-N'-(3,4-dichlorophenyl)urea, 2,4,4'-trichloro-2'-hydroxydiphenyl ether (triclosan), 4-chloro-3,5-dimethylphenol, 2,2'-methylenebis(6-bromo-4-chlorophenol), 3-methyl-4-(1-methylethyl)phenol, 2-benzyl-4-chlorophenol, 3-(4-chlorophenoxy)-1,2-propanediol, 3-iodo-2-propynyl butylcarbamate, chlorohexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, for example n-octylsalicylamide or n-decylsalicylamide.

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen® CAT). The substances inhibit enzyme activity, thereby reducing the formation of odor. Other substances which are suitable esterase inhibitors are sterol sulfates or phosphates, for example lanosterol, cholesterol, campesterol, stigmasterol and sitosterol sulfate or phosphate, dicarboxylic acids and esters thereof, for example glutaric acid, monoethyl glutarate, diethyl glutarate, adipic acid, monoethyl adipate, diethyl adipate, malonic acid and diethyl malonate, hydroxycarboxylic acids and esters thereof, for example citric acid, malic acid, tartaric acid or diethyl tartrate, and zinc glycinate.

Suitable odor absorbers are substances which are able to absorb and largely retain odor-forming compounds. They lower the partial pressure of the individual components, thus also reducing their rate of diffusion. It is important that in this process perfumes must remain unimpaired. Odor absorbers are not effective against bacteria. They comprise, for example, as main constituent, a complex zinc salt of ricinoleic acid or specific, largely odor-neutral fragrances which are known to the person skilled in the art as "fixatives", for example extracts of labdanum or styrax or certain abietic acid derivatives. The odor masking agents are fragrances or perfume oils, which, in addition to their function as odor masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal raw materials, for example civet and castoreum. Typical synthetic fragrance compounds are products of the ester, ether, aldehyde, ketone, alcohol and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Ethereal oils of relatively low volatility, which are mostly used as aroma components, are also suitable as perfume oils, e.g. sage oil, camomile oil, oil of cloves, melissa oil, mint oil, cinnamon leaf oil, linden flower oil, juniper berry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Antiperspirants reduce the formation of perspiration by influencing the activity of the eccrine sweat glands, thus counteracting underarm wetness and body odor. Aqueous or anhydrous formulations of antiperspirants typically comprise one or more of the following ingredients: astringent active ingredients, oil components, nonionic emulsifiers, coemulsifiers, bodying agents, auxiliaries, for example thickeners or complexing agents, and/or nonaqueous solvents, for example ethanol, propylene glycol and/or glycerol.

Suitable astringent antiperspirant active ingredients are primarily salts of aluminum, zirconium or of zinc. Such suitable antihydrotic active ingredients are, for example, aluminum chloride, aluminum chlorohydrate, aluminum dichlorohydrate, aluminum sesquichlorohydrate and complex compounds thereof, e.g. with 1,2-propylene glycol, aluminum hydroxyallantoinate, aluminum chloride tartrate, aluminum zirconium trichlorohydrate, aluminum zirconium tetrachlorohydrate, aluminum zirconium pentachlorohydrate and complex compounds thereof, e.g. with amino acids, such as glycine. In addition, customary oil-soluble and water-soluble auxiliaries may be present in antiperspirants in relatively small amounts. Such oil-soluble auxiliaries may, for example, be anti-inflammatory, skin-protective or perfumed ethereal oils, synthetic skin-protective active ingredients and/or oil-soluble perfume oils.

Customary water-soluble additives are, for example, preservatives, water-soluble fragrances, pH regulators, e.g. buffer mixtures, water-soluble thickeners, e.g. water-soluble natural or synthetic polymers, for example xanthan gum, hydroxyethylcellulose, polyvinylpyrrolidone or high molecular weight polyethylene oxides.

Film formers that can be used are described in the following text. Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof, and similar compounds.

Suitable antidandruff active ingredients are piroctone olamine (1-hydroxy-4-methyl-6-(2,4,4-trimethylpentyl)-2-(1H)-pyridinone monoethanolamine salt), Baypival® (climbazole), Ketoconazole®, (4-acetyl-1-{-4-[2-(2,4-dichlorophenyl) r-2-(1H-imidazol-1-ylmethyl)-1,3-dioxylan-c-4-ylmethoxyphenyl}piperazine, ketoconazole, elubiol, selenium disulfide, colloidal sulfur, sulfur polyethylene glycol sorbitan monooleate, sulfur ricinol polyethoxylate, sulfur tar distillates, salicyclic acid (or in combination with hexachlorophene), undecylenic acid monoethanolamide sulfosuccinate Na salt, Lamepon® UD (protein undecylenic acid condensate), zinc pyrithione, aluminum pyrithione and magnesium pyrithione/dipyrithione magnesium sulfate.

The swelling agents for aqueous phases may be montmorillonites, clay mineral substances, Pemulen, and alkyl-modified Carbopol grades (Goodrich).

Suitable insect repellents are N,N-diethyl-m-toluamide, 1,2-pentanediol or ethyl butylacetylaminopropionate.

To improve the flow behavior, hydrotropes, for example ethanol, isopropyl alcohol, or polyols, can be used. Polyols which are suitable here preferably have 2 to 15 carbon atoms and at least two hydroxyl groups. The polyols can also contain further functional groups, in particular amino groups, or be modified with nitrogen. Typical examples are:
- glycerol;
- alkylene glycols, for example, ethylene glycol, diethylene glycol, propylene glycol, butylene glycol, hexylene glycol, and polyethylene glycols with an average molecular weight of from 100 to 1 000 daltons;
- technical-grade oligoglycerol mixtures with a degree of self-condensation of from 1.5 to 10, for example, technical-grade diglycerol mixtures with a diglycerol content of from 40 to 50% by weight;
- methylol compounds, such as trimethylolethane, trimethylolpropane, trimethylolbutane, pentaerythritol and dipentaerythritol;
- lower alkyl glucosides, in particular those with 1 to 8 carbon atoms in the alkyl radical, for example methyl and butyl glucoside;
- sugar alcohols with 5 to 12 carbon atoms, for example sorbitol or mannitol,
- sugars with 5 to 12 carbon atoms, for example glucose or sucrose;
- amino sugars, for example glucamine;
- dialcohol amines, such as diethanolamine or 2-amino-1,3-propanediol.

Suitable preservatives are, for example, phenoxyethanol, formaldehyde solution, parabenes, pentanediol or sorbic acid, and the other classes of substance listed in Annex 6, Part A and B of the Cosmetics Directive.

Perfume oils which may be used are preferably mixtures of natural and synthetic fragrances. Natural fragrances are extracts from flowers (lily, lavender, rose, jasmine, neroli, ylang-ylang), stems and leaves (geranium, patchouli, petitgrain), fruits (aniseed, coriander, cumin, juniper), fruit peels (bergamot, lemon, orange), roots (mace, angelica, celery, cardamom, costus, iris, calmus), woods (pine wood, sandalwood, guaiac wood, cedarwood, rosewood), herbs and grasses (tarragon, lemon grass, sage, thyme), needles and branches (spruce, fir, pine, dwarf-pine), resins and balsams (galbanum, elemi, benzoin, myrrh, olibanum, opoponax). Also suitable are animal raw materials, for example civet and castoreum. Typical synthetic fragrance compounds are products of the ester, ether, aldehyde, ketone, alcohol and hydrocarbon type. Fragrance compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethylmethylphenyl glycinate, allyl cyclohexylpropionate, styrallyl propionate and benzyl salicylate. The ethers include, for example, benzyl ethyl ether, the aldehydes include, for example, the linear alkanals having 8 to 18 carbon atoms, citral, citronellal, citronellyloxyacetaldehyde, cyclamen aldehyde, hydroxycitronellal, lilial and bourgeonal, and the ketones include, for example, the ionones, α-isomethylionone and methyl cedryl ketone, the alcohols include anethole, citronellol, eugenol, isoeugenol, geraniol, linalool, phenylethyl alcohol and terpineol, and the hydrocarbons include predominantly the terpenes and balsams. Preference is, however, given to using mixtures of different fragrances which together produce a pleasing fragrance note. Ethereal oils of relatively low volatility, which are mostly used as aroma components, are also suitable as perfume oils, e.g. sage oil, camomile oil, oil of cloves, melissa oil, mint oil, cinnamon leaf oil, linden blossom oil, juniper berry oil, vetiver oil, olibanum oil, galbanum oil, labolanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Dyes which can be used are the substances which are approved and suitable for cosmetic purposes. These dyes are normally used in concentrations of from 0.001 to 0.1% by weight, based on the total mixture.

### Examples

### Example 1: preparation of an active ingredient

352 kg of water has been heated to 85 °C and 37.4 kg of Cassia alata leaves have been added. This mixture has been stirred for 2 hours at 85 °C. After cooling to room temperature, passing through a tamis, centrifugation and filtration, further components have been added (see list below), water has been evaporated at 80 °C under vacuum, the mixtured has been cooled to room temperature. Finally, a final filtration has been carried out (K150, size of 2-4 µm).

The INCI name of the active ingredient is: Water (and) Glycerin (and) Cassia alata leaf extract.

The composition is (% means % by weight):

| | |
|---|---|
| Glycerol | 60 % |
| Cassia alata leaf extract | 4.15 % |
| Chlorphenesin | 0.4 % |
| Methylparaben | 0.2 % |
| Water | 35.25 % |

The dose of use of this active ingredient in the compositions of example 4 and subsequent examples has been 1 to 3 % by weight.

### Example 2: preparation of the extract of Senna alata (= Cassia alata)

The extract of Senna alata is prepared as described in example 1 except that the further components have not been added and that the extract has been dried (freeze-drying or atomization).

### Example 3: preparation of the main molecule of the active ingredient

In the active ingredient according to example 1 kaempferol 3-O-sophoroside has been identified as a tracer molecule (HPLC at 350 nm, main peak). This molecule has been purified by HPLC and low pressure chromatography and its structure has been determined by ¹H-NMR (Nuclear Magnetic Resonance), ¹³C-NMR and ESI MS (ElectroSpray Ionization Mass Spectrometry) (degree of purity > 90 %).

The CAS number of this main molecule of the active ingredient is: 19895-95-5.

### Example 4: effect of the active ingredient of example 1 on the level of gadd45α and gadd45γ proteins with or without UV-B irradiation

### 1. Materials and methods

### Reagents:

Polyclonal antibodies anti-Gadd45α, anti-Gadd45γ have been obtained from Santa Cruz Biotechnology and the secondary antibody biotine-conjugated from Clinisciences. The FITC-conjugated streptavidine has been obtained from Amersham Biosciences. PBS (Phosphate buffered saline-pH 7.2) and Evans blue were obtained from BioMérieux.

### Human primary keratinocyte cultures:

Keratinocyte cell suspensions have been prepared by standard digestion by trypsine of human adult epidermis specimen collected from plastic surgery. Keratinocytes have been seeded on glass slides in an incubation chamber and have been grown in a culture medium until confluence.

### Treatment with the active ingredient and UV-B irradiation:

When confluent cultures have been obtained, the medium was changed by a balanced salt solution containing the active ingredient at a concentration of 3 % by weight. Control experiments without addition of the active ingredient have been carried out. The keratinocytes have then been irradiated with UV-B at a rate of 30 mJ/cm². Control experiments have also been carried out without UV-B irradiation. After various post-radiation incubation times, the Gadd45α and Gadd45γ protein levels have been evaluated on glass slides by immunocytochemistry.

### Immunocytochemistry:

Keratinocyte cultures on glass slides have been dried at 37 °C. They have then been fixed in cold methanol for 10 minutes and washed in PBS (phosphate buffered saline). Keratinocyte cultures have been incubated overnight at 4 °C using the polyclonal antibodies raised against Gadd45α or Gadd45γ at a dilution of 1/100.
Keratinocyte cultures have then been incubated with a biotine-conjugated anti-rabbit antibody IgG for 45 minutes at a dilution of 1/100 then with fluorescein isothiocyanate (FITC)-conjugated streptavidine for 45 minutes at a dilution of 1/30. The different incubations have been separated by extensive washing with PBS. Negative controls have been produced by omission of the primary antibody. After extensive washing with PBS, immunostained keratinocytes have been counterstained for 10 minutes with Evans blue and have been mounted on coverslip. Immunostaining has been observed under confocal laser scanning microscope (a Zeiss microscope has been used).

### Quantification of immunostaining:

Pictures obtained with a confocal laser scanning microscope have been converted in colour numeric images and analysed using a mathematical morphology software (Quantimet Q500, Leica). Results have been expressed in percentage of area occupied by gadd45α and gadd45γ proteins (FITC) in the keratinocyte culture.

### 2. Results

Without UV-B irradiation, a small rate of Gadd45α protein has been detected in non-treated keratinocytes. This rate has been maintained constant during all the incubation period (1.6 % and 1.9 % for 30 and 240 minutes incubation, respectively). The application of the active ingredient has highly increased the rate of Gadd45α, that was maintained at a high level during the incubation period (7.8 % and 8.5 % for the 2 different incubation times).

As described in the literature, the UV-B irradiation has increased the rate of Gadd45α protein in keratinocytes. This rate increase has been observed after a short post-UV incubation (3.6 % after 30 minutes) and has been more pronounced after a long post-UV incubation (5.3 % after 240 minutes). The treatment of keratinocytes with the active ingredient of example 1 highly increased the rate of Gadd45α protein detected after UV-B irradiation (5.8 % and 12.9 % after 30 minutes and 240 minutes of post-UV incubation).

Without UV-B irradiation, a significant rate of Gadd45y protein has been detected in non-treated keratinocytes. This rate decreased during the incubation period (from 9.1 % to 3.4 % for 30 to 240 minutes of incubation time). The application of the active ingredient of example 1 has highly and quickly increased the rate of Gadd45y. Thirty minutes after application of the active ingredient, the rate of Gadd45y protein has increased to 24.9 % (compared to 9.1 % without treatment). The active ingredient has maintained an increased rate of Gadd45y protein during all of the incubation time (15.2 % compared to 3.4 % in the control at 240 minutes post-UV), even when the expression has been slightly decreased between 30 and 240 minutes.
The UV-B irradiation did not increase the rate of Gadd45y protein after the short 30 minutes post-UV incubation time. But after a longer period, a significant rise of Gadd45y protein level has been observed (21.0 % compared to 3.4 % without UV). The kinetic of Gadd45y induction by UV-B might be longer than those observed for the *gadd45α* gene. The treatment by the active ingredient has strongly risen the rate of Gadd45y protein compared to untreated condition. This increase has been clear at 30 minutes post-UV (19.1 % versus 8.1 %) and has been maintained for 240 minutes after UV irradiation (46.7 % compared to 21.0 %).

| | Without UV-B irradiation | | | | With UV-B irradiation | | | |
|---|---|---|---|---|---|---|---|---|
| Post UV incubation period | 30 minutes | | 240 minutes | | 30 minutes | | 240 minutes | |
| Treatment with the active ingredient prepared according to example 1 | NO control | YES | NO control | YES | NO control | YES | NO control | YES |
| % of Gadd45α protein level occupation in the keratinocyte culture | 1.6 | 7.8 | 1.9 | 8.5 | 3.6 | 5.8 | 5.3 | 12.9 |
| % of Gadd45γ protein level occupation in the keratinocyte culture | 9.1 | 24.9 | 3.4 | 15.2 | 8.1 | 19.1 | 21.0 | 46.7 |

These results show that the active ingredient can induce the level of Gadd45α and Gadd45y proteins with or without UV-B irradiation This increases the efficiency of the DNA repair systems of the cells.

### Example 5: effect of the active ingredient on the gadd45α gene expression

### 1. Material and methods

### Human primary keratinocyte cultures:

Keratinocyte cell suspensions have been prepared by standard digestion by trypsine of human adult epidermis specimen collected from plastic surgery. Keratinocytes have been seeded on glass slides in an incubation chamber and grown in a culture medium until confluence.

### Treatment with the active ingredient according to example 1, with the extract of Senna alata leaves and with kaempferol 3-O-sophoroside and UV-B irradiation:

When confluent cultures have been obtained, the medium has been changed by a balanced salt solution containing the active ingredient at a concentration of 1 %, the Senna alata leaves extract at 0.1 % and the kaempferol 3-O-sophoroside at 0.1 %. A control experiment without addition of the active ingredient has been carried out. After 90 or 180 minutes of incubation time, the RNA was extracted from the different cultures. The concentration of RNA and the purity have been estimated by optical densities at 260 and 280 nm.

### Real time RT-PCR analysis (SEREWKO MM., POPA C., DAHLER AL., SMITH L., STRUTTON GM., COMAN W., DICKER AJ. SAUNDERS NA.: Alterations in Gene Expression and Activity during Squamous Cell Carcinoma Development, Cancer Re search, 2002, volume 62 number 13, pages 3759 to 3765):

Quantitative analysis has been carried out using the LightCycler™ software using a real-time fluorogenic detection. PCR product specificity has been verified with a melting curve procedure. Detection and quantification of the house keeping gene, actin, has been carried out as a control for RT-PCR quality. The results are expressed as a ratio between the measured gadd45α and the actin cDNA levels. Each experiment has been repeated in three independent assays.

### 2. Results

| | Incubation for 90 minutes (average +/-SD of 3 assays) | Incubation for 180 minutes (average +/-SD of 3 assays) |
|---|---|---|
| Control medium | 0.37 +/- 0.03 | 0.39 +/- 0.05 |
| Treatment with the active ingredient prepared according to example 1 | 0.51 +/- 0.10* | 0.50 +/- 0.09* |
| Treatment with the plant extract prepared according to example 2 | 0.55 +/- 010* | 0.57 +/- 0.10* |
| Treatment with kaempferol 3-O-sophoroside (example 3) | 0.48 +/- 0.050* | 0.50 +/- 0.14 |
| Statistics: student t test *: = p < 0.05 | | |

The ratio of *gadd45α* gene expression (versus house keeping gene) has not changed between 90 and 180 minutes of incubation at 37 °C. This result shows that the active ingredient can quickly induce the level of mRNA without stress to pre-condition the cells. The extract of the leaves of the plant Senna alata and also kaempferol 3-O-sophoroside has induced the level of mRNA without stress.

This pre-conditioning allows the cells to rise the DNA repair efficiency after stress.

## Claims

1. The use of an active ingredient that increases gadd45 gene expression in mammalian skin (preferably human skin) for the manufacture of a cosmetic composition or for the cosmetic treatment of human skin and/or hair.

2. The use according to claim 1, wherein gadd45 gene expression is the expression of at least one gene selected from the group consisting of gadd45α, gadd45β and gadd45γ.

3. The use according to claim 1 or 2, wherein the active ingredient is an aqueous extract of the leaves of the plant Cassia alata.

4. The use according to claim 3, wherein the aqueous extract of the leaves of the plant Cassia alata is obtainable by extracting the leaves of the plant Cassia alata with water and removing the water in order to obtain the extract.

5. The use according to claim 1 or 2, wherein the active ingredient is selected from the group consisting of derivatives of kaempferol (such as kaempferol 3-O-sophoroside), lignans, tannins (such as catechuic tannins), retinoids, derivatives of retinoid, vitamin D and derivatives of vitamin D.

6. The use according to any of claims 1 to 5, wherein the active ingredient is used in a composition comprising the active ingredient and at least one cosmetic adjuvant.

7. The use according to claim 6, wherein the cosmetic adjuvant is selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

8. The use according to any of claims 1 to 7, wherein the cosmetic treatment of human skin comprises the protection of the skin against ageing or against photoageing or against UV radiation or against pollutants or against IR radiation or against stress in general or against inflammation or against irritation or against sensitization or against a weakening of the immune system.
